# EUROPEAN PATENT SPECIFICATION

(11) **EP 1 194 170 B1**
(45) Date of publication and mention of the grant of the patent: **20.02.2008**
(21) Application number: 00946088.2
(22) Date of filing: 11.07.2000
(51) Int. Cl.: A61K 9/00, A61K 47/26, A61K 38/27

(54) **GROWTH HORMONE FORMULATIONS**
FORMULIERUNGEN VON WACHSTUMSHORMONEN
PREPARATIONS D'HORMONES DE CROISSANCE

(30) Priority: 12.07.1999 GB 9916252; 12.08.1999 GB 9918902
(43) Date of publication of application: 10.04.2002
(73) Proprietor: SANDOZ AG, 4002 Basel (CH)
(72) Inventor: SIEBOLD, Bernhard, D-6250 Kundl (AT); STEVENS, John, CH-1227 Carouge (CH)
(74) Representative: de Weerd, Petrus G.W.
(86) International application number: PCT/GB2000/002664
(87) International publication number: WO 2001/003741

(56) References cited:
- WO-A-94/03198
- WO-A-97/07816
- WO-A-97/29767
- US-A- 5 096 885
- US-A- 5 126 324
- US-A- 5 567 677
- US-A- 5 610 134

## Description

The present invention relates to liquid formulations of growth hormone (GH) suitable for administration to the human or animal body. More particularly, the invention relates to liquid formulations of human growth hormone (hGH) which are pharmaceutically more acceptable and preferable and yet can be subjected to a variety of manufacturing process steps without appreciable loss in activity or appreciable loss of stability.

Native hGH is a single polypeptide chain protein consisting of 191 amino acids. The protein is internally cross-linked by two disulphide bridges and in monomeric form exhibits a molecular weight of 22kDa. GH of animal species is closely homologous in amino acid sequence to that of humans and is therefore very similar in its characteristics.

A major biological effect of GH is to promote growth throughout a range of organs and tissues in the body. GH responsive organs or tissues include the liver, intestine, kidneys, muscles, connective tissue and the skeleton.

Hypopituitary dwarfism is a condition which is readily treated by administering GH to a subject suffering the condition. Prior to the production of large quantities of hGH by recombinant means only limited amounts of hGH could be prepared by laborious extraction of pituitary glands from human cadavers. This practice carried with it risks associated with infectious agents, eg the agent responsible for Creutzfeldt-Jakob disease (CJD), and that these agents might be passed to the patient receiving GH. The isolation of the hGH gene and the construction of transformed host cells expressing hGH in cell culture has opened up not only a more reliable, safer and more cost effective treatment of hypopituitary dwarfism, but the possibility of using hGH for treatment of other diseases and conditions as well.

A long appreciated problem with aqueous liquid formulations of pharmaceutical proteins, not just hGH, has been that of Instability during storage over a period of time. hGH in aqueous solution is known to undergo a variety of degradative

changes. Chemical changes such as deamidation occur and this may be related to the pH of the solution during storage. Oxidation of methionine residues may occur. There is also the possibility of a clipping of the peptide backbone occurring due to hydrolysis reactions. Also there are physical changes which may include aggregation for example resulting in the formation of insolubles.

An early suggestion of how to deal with the problems of instability noted above was freeze drying but this of course meant that the resulting lyophilised product needed reconstitution immediately or shortly prior to administration. In the circumstances of routine self-administration by a patient at home, this normally means that the patient has the task of reconstituting the lyophilised preparation into an aqueous solution. This is inconvenient for the patient and carries with it a risk of improper reconstitution due to lack of care, lack of attention to detail and instructions or simply misunderstanding.

US 4 968 299 (Kabi Pharmacia) describes a device for a patient to use to perform reconstitution of a lyophilised preparation thereby seeking to lessen the possibility of errors in reconstitution. Even so, the need for reconstitution itself is inconvenient for a patient and the reconstituted hGH is only stable for 3 weeks when stored at 2-8°C. Effective administration by the patient over a period of months still therefore required careful attention to detail and instructions and so there were still serious risks of non-compliance in the treatment regime.

In any event, freeze drying has the disadvantage of being a costly and time consuming manufacturing step.

Efforts to simplify seff-administration for patients have therefore focused on ways of providing sufficiently stable aqueous hGH formulations in a ready to use form.

Protein instability in aqueous solution was appreciated to be a general phenomenon, not one associated particularly with hGH.

EP-A-0 131 864 (Hoechst Aktiengesellschaft) describes the prevention of aggregation in proteins of greater than 8.5 kDa in aqueous solution by using surfactants.

EP-A-0 211 601 (International Minerals & Chemical Corporation) although perhaps primarily concerned with sustained release formulations describes how GH can be stabilised in solution as a liquid by formulating it with non-ionic surfactants, in particular certain polyoxyethylene-polyoxypropylene block copolymers, eg PLURONIC (trade mark of BASF) or GENAPOL (trade mark of Hoechst) block copolymer.

WO 94/03198 (Genentech) is another disclosure following the previous teachings about using non-ionic surfactant as an hGH stabiliser in liquid formulations. The range 0.1-5% (w/v) non-ionic surfactant in the formulation is said to permit the formulation to be exposed to shear and surface stresses without causing denaturation of the GH protein. In particular, the surfactant-containing formulations are seen as being useful in pulmonary dosing and needleless jet injector guns.

However, surfactants are toxic substances, and their use should be avoided or at least minimised so far as is possible. This is especially so where formulations are to be administered daily or very frequently, particularly where children and chronic treatments are concerned.
A variety of other ways of stabilising aqueous hGH formulations have been proposed. WO 89/09614 (Genentech) teaches a formulation of hGH comprising glycine, mannitol and a buffer, there being an hGH:glycine molar ratio of from 1:50 to 1:200.

EP-A-0 303 746 (International Minerals and Chemical Corporation) teaches that aqueous GH may be stabilised by formulating it with a polyol, eg non-reducing sugars, sugar alcohols, sugar acids, lactose, pentaerythritol, water-soluble dextrans and Ficoll; an amino acid, eg glycine, arginine and betaine; an amino acid polymer having a charged side group of physiological pH; and finally a choline derivative, eg choline chloride, choline dihydrogen citrate or dicholine mucate. Many of the polymeric materials referred to above may carry some risk In administration to patients. Pharmaceutical regulatory requirements dictate that any unnecessary additives, particularly synthetic additives (eg pentaerythritol) must be avoided in order to reduce risks to patients. Many of the suggested stabilisers in the disclosure would not appear clinically acceptable and therefore would not enable a pharmaceutically acceptable formulation to be made.

WO 92/17200 (Genentech) is concerned with stabilising hGH, not just in liquid but also in lyophilised preparations. The suggestion is that stable zinc:hGH dimers are produced. The zinc:hGH dimers are made up of two zinc ions and two hGH molecules.

WO 93/12811 (Novo Nordisk) discloses a liquid hGH formulation in which asparagine is used as the stabilising and buffering substance,

WO 93/19776 (Kabi Pharmacia) teaches the totally unexpected finding that when an aqueous hGH product is formulated with citrate buffer then it is more stable than when it is formulated with phosphate buffer.

An object of the present invention is to provide a sufficiently stable hGH formulation instantly usable by patients without the need for any particular preparation or reconstitution procedures. Another object of the invention is to provide a formulation which can be stored at home in a domestic refrigerator for at least a few months. Yet another object of the invention is to provide a bulk liquid formulation which can be dispensed and filled into cartridges for patient use without unacceptable losses in GH activity or unacceptable instability, in particular without unacceptable aggregation occurring. A still further object of the invention is to provide a sufficiently stable liquid formulation which avoids or minimizes the use of pharmaceutically unacceptable or undesirable components, in other words to provide an even more pharmaceutically acceptable formulation.

A yet further object of the invention is to provide liquid formulations which avoid the problem of crystal formation when stored in the refrigerator for long periods, e.g. up to 6 or 18 months, or if stored for periods of time outside a refrigerator, e.g. periods of several days, weeks or months.

Entirely contrary to the existing wisdom in the art, the present inventors have surprisingly discovered that it is not actually necessary to employ a variety of additional stabilising agents in solution above and beyond simply hGH and a phosphate buffer In order to achieve the aforementioned objectives. Furthermore, the present invention arises in the face of the prior art teachings about how surfactants are essential for stability of aqueous solutions of GH and also how phosphate buffered solutions fall to give good stability compared to citrate buffer.

Advantageously, the aforementioned formulations lacking preservative when stored in ampoules provide a convenient way of presenting single shot dosages. For multi-shot dosages the presence of a preservative is preferable.

A hitherto unappreciated and Indeed surprising advantage of all of the aforementioned formulations is that they are storage stable at refrigeration temperatures in the range 2-8°C. A variety of test procedures can be used to assess the stability of formulations over time. Representative examples of test procedures are given in Example 3 herein and also in WO 94/03198 but these procedures are in no way exhaustive or comprehensive of the tests which can be employed to assess stability.

The filling of dosage containers with growth hormone formulations lacking any non-ionic surfactant and using commercial available filling apparatus has been found to result in unacceptable levels of aggregation of growth hormone. However, provided that the fluid pressures and shear stresses are minimised during filling procedures (whether using commercial filling apparatus or not) then surfactant levels can be minimised or dispensed with altogether. The actual balance required to be achieved between physical filling stresses and the concentration of surfactant is a matter for routine empirical determination by one of average skill in the art.

Depending on the levels of physical stresses or shear forces arising during filling and where a non-ionic surfactant is needed to avoid significant aggregation then the concentrations of non-ionic surfactant may be as low as about 0.2% (w/v), usually less than 0.05% (w/v), preferably less than 0.04% (w/v), more preferably less than 0.01% (w/v), or even more preferably less than 0.001% (w/v).

Non-ionic surfactants may include a polysorbate, such as polysorbate 20 or 80, etc., and the poloxamers, such as poloxamer 184 or 188, Pluronic® polyols, and other ethylene/polypropylene block polymers.

Unexpectedly, the inventors have found that phosphate buffer may be used in GH formulations and it is surprisingly good at stabilising the resultant formulations, either during processing such as filling containers, or during storage.

An absence or use of only a very low concentration of non-ionic surfactant has also surprisingly been found not to adversely affect the stability of GH formulation stored in containers at refrigeration temperatures (in the range 2-8°C for example). Storage for at least three months and longer to at least 6 months or 12 months is possible without unduly affecting the efficacy or pharmaceutical acceptability of the GH formulations.

In a one aspect the invention provides a liquid growth hormone formulation comprising growth hormone in phosphate buffered solution as defined in claim 1.

In the aforementioned aspect of the invention, the phosphate buffered solution is isotonic. The isotonicity may be provided by a neutral sall, eg NaCl; or monosaccharide, eg lactose; a disaccharide eg sucrose or a sugar alcohol, eg mannitol.

The inventors have also found that certain compounds can be used advantageously in place of neutral salt in order to render the GH formulations isotonic.

Thus, in a further aspect the invention provides a liquid growth hormone formulation as defined in claim 1, the compound conferring isotonicity being selected from one or more of monosaccharides, eg lactose; disaccharides, eg sucrose; sugar alcohols, eg mannitol

As to the pH, the formulations fall within the range pH 6.15 to 6.5.

Surprisingly, and for all formulations described herein, the inventors have found that the problem of crystallisation in formulations can be avoided or minimised by ensuring a pH of about 6.2 or greater.

Preferably the pH of the formulations is in the range 6.2 to 6.5 to avoid or minimise crystallisation.

Therefore the invention includes liquid formulations as described herein having no detectable crystallisation on storage. The storage may be at least one month, preferably six weeks, more preferably a period in the range of about 1 month to 4 month, most preferably 3 months. The storage temperature may be about 2°C or greater, preferably about 4°C or greater, more preferably a temperature in the range from about 2°C to less than 40°C, even more preferably a temperature in the range from about 2°C to 25°C, most preferably 15°C.

The crystallisation is preferably that of growth hormone. Preferably any crystallisation in the liquid formulation is detected directly by eye, more preferably under the light microscope at 5x magnification, even more preferably under the light microscope at 10x magnification. Prior to observation under the light microscope formulations may be filtered and the presence or absence of crystals on the filter determined. When viewing under the light microscope the filter may have a pore size of about 5µm.

A particularly preferred test for crystallisation is to store the formulation for 3 months at 15°C and observe the presence or absence of crystals by eye.

As to a preservative this is preferably selected from one or more of phenol, benzyl alcohol, meta-cresol, methyl paraben, propyl paraben and benzalkonium chloride although any other preservative or antibacterial compound may be used at an appropriate concentration such that the formulation remains pharmaceutically acceptable.

In preferred embodiments, the phosphate buffered solution is made up of appropriate amounts of appropriate hydrated forms of NaH₂PO₄ and Na₂HPO₄ needed to achieve the desired concentration and pH of buffer, as will be readily recognised and known by one of average skill in the art.

The growth hormone is human.

In especially preferred formulations, the growth hormone exhibits less than 0.01% aggregation, preferably less than 0.1%, more preferably less than 1%, even more preferably less than 10% aggregation. The aggregation may be measured by the standard size exclusion HPLC test referred to in more detail later but any suitable method of measuring aggregation can be employed.

The invention also includes devices for administering a liquid to a subject by injection and loaded for use with at least one dosage unit of any of the liquid growth hormone formulations hereinbefore described. An example of such a device is a pen injector device. The subject is preferably a human.

Also provided by the invention are kits comprising an injection device and separate container of any of the liquid growth hormone formulations as hereinbefore described. The container is preferably adapted to engage with the injection device such that in use the liquid formulation in the container is in fluid connection with the outlet of the injection device.

In particularly preferred embodiments the injection device is a pen injector and the container is a cartridge.

Furthermore, the Invention provides a cartridge containing any of the liquid formulations as hereinbefore described for use with a pen injector device.

Another surprising discovery made by the inventors is that if containers of GH are filled and closed so that there is no airspace or access to the air then not only is sterility of the contents of the containers more reliably assured but that this factor too contributes to minimising or avoiding aggregation of GH.

Thus, a still further aspect of the invention includes sealed containers of liquid GH formulations in which there is substantially no airspace in the filled containers.

Particularly preferred human growth hormone is produced by recombinant means, for example as taught in EP-A-0 217 822 (SCIOS NOVA). Variants of human growth hormone which may be used in accordance with the invention, alone or in combination with one another and the native hormone include the 191 amino acid species known as somatropin and the 192 amino acid N-terminal methionine (met) species known as somatrem. There is also the variant known as hGH-V found naturally in the placenta during pregnancy and for which the gene is known and recombinant protein has been prepared.

The amount of hGH in the liquid formulation of the invention depends on the volume of the formulation and the number of doses of hGH that volume is intended to provide. A preferred dosage volume is 0.4ml but volumes in the range 0.01ml to 1.0ml may be used. Other preferred dosage volumes may fall in the range 0.1ml to 0.6ml.

In a preferred unit dosage for daily administration the amount of hGH administered is 1.3mg although the precise dosage amount may vary depending on the particular individual. Dosage amounts in the range 0.033mg to 3.33mg hGH may be employed, preferably dosages in the range 0.33mg to 2.0mg. Increased dosage amounts are appropriate where the frequency of administration is reduced.

The volumes and/or dosage amounts may vary from individual to individual in accordance with specific advice from the clinician in charge.

Usually, formulations in accordance with the invention may comprise hGH in the range 0.5mg/ml to 20mg/ml, preferably 1mg/ml to 15mg/ml, more preferably 2mg/ml to 10mg/ml, even more preferably 3mg/ml to 5mg/ml.

The invention also includes kits comprising an injection device and a separate container of liquid growth hormone formulation as hereinbefore described. When the administration device is simply a hypodermic syringe then the kit may comprise the syringe, a needle and a vial or ampoule containing the hGH formulation for use with the syringe. In more preferred embodiments the injection device is other than a simple hypodermic syringe and so the separate container is adapted to engage with the injection device such that in use the liquid formulation in the container is in fluid connection with the outlet of the injection device.

Examples of administration devices include hypodermic syringes and pen injector devices,

Particularly preferred injection devices are the pen injectors in which case the container is a cartridge, preferably a disposable cartridge.

In another aspect the invention provides a cartridge containing a liquid growth formulation as hereinbefore described for use with a pen injector device. The cartridge may contain a single dose or multiplicity of doses of growth hormone.

Preferred embodiments of the invention will now be described by way of the following examples with reference to drawings in which:
Figure 1 is a plot of comparative stability data at 2-8°C for hGH formulations additionally containing phosphate buffer at pH 5.6, sodium chloride and benzyl alcohol. The comparison is of these formulations with and without Pluronic surfactant. Time in weeks is plotted against log% purity of hGH.
Figure 2 is a plot of comparative stability data at 2-8°C for hGH formulations additionally containing sodium chloride and benzyl alcohol at pH 6.0. The comparison is of these formulations containing citrate or phosphate buffer. Time in weeks is plotted against log% purity of hGH.
Figure 3 is a plot of comparative stability data at 2-8°C for hGH formulations. The comparison is between hGH formulations containing isotonic citrate buffer and Pluronic surfactant with hGH formulations containing just isotonic phosphate buffer and no surfactant.

### Example 1 - Preparation and purification of bulk recombinant hGH

Recombinant hGH is produced in cell cultures of CHO cells transformed with the hGH gene to express the hGH protein under culture conditions. Details of how the cells are made and grown are described in EP-A-0 217 822 (SCIOS NOVA). The modification of culture conditions for the growth of cultures on an industrial or commercial scale is well within the abilities of one of average skill in the art.

Once produced by the cells in culture the hGH needs to be extracted and purified into a form suitable for pharmaceutical use. This is carried out according to the procedures described in AU 629177 (University of New South Wales & Garvan Institute of Medical Research).

### Example 2- Preparation of stable liquid formulation

Bulk formulation is prepared by mixing the various components together. The order of mixing of components is not critical. Also, the precise state or form of the various components immediately prior to mixing is not critical either. In preferred ways of preparing the formulation the components are prior to mixing in the most convenient state for mixing and the order and mode of mixing is also selected to be the most convenient. Particularly preferred examples of formulations are given below:

### Reference Formulation I

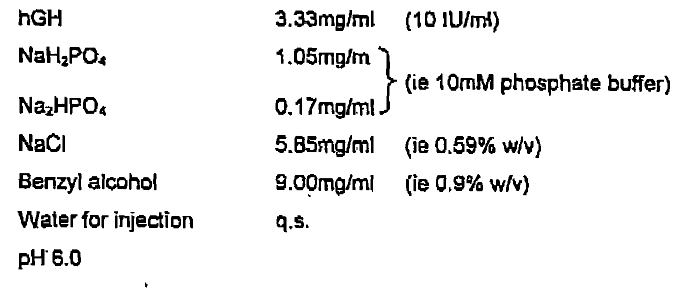

### Reference Formulation II

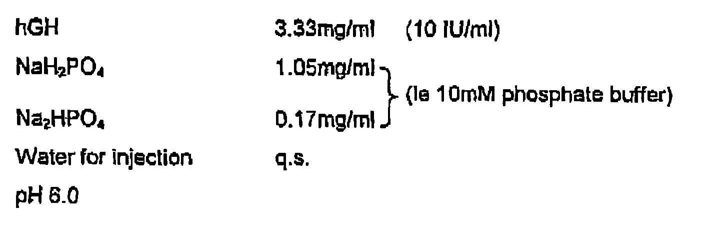

### Reference Formulation III

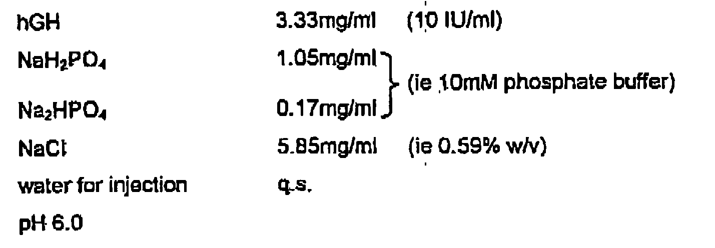

### Reference Formulation IV

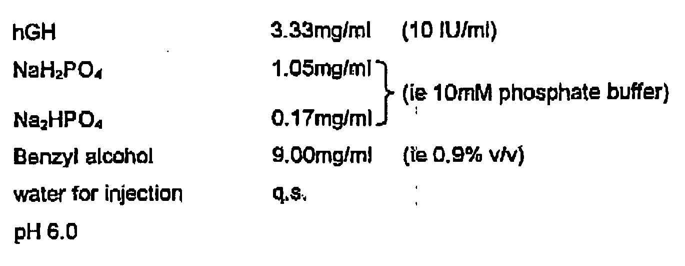

### Reference Formulation V

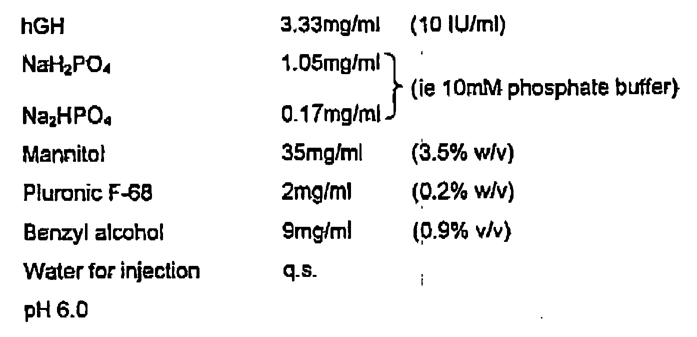

### Formulation VI

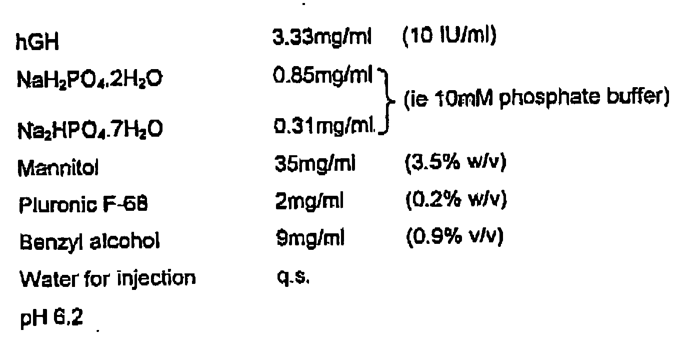

The above exemplified formulations were prepared as follows:
1. A double strength excipient solution is prepared by dissolving all the required excipients in water for injection, and adjusting the pH to that required using molar hydrochloric acid or sodium hydroxide solutions.
2. The bulk growth hormone solution is placed in a vessel and the excipient solution added with careful stirring.
3. The pH is readjusted if necessary, and the solution made to final volume For the filling of cartridges for use with pen injectors the solution is filtered through a sterilising filter and filled into injection cartridges sealed at one end with a moveable plunger, and at the other with an aluminium seal containing a rubber septum.

Other test formulations were prepared generally in this way and details of these formulations are given in the example below.

### Example 3 - Testing for stability of aqueous hGH formulation

Samples of the product were stored under controlled conditions at 2-8°C, and analysed at various time points The stability of the product was determined by the use of two HPLC methods, both according to the European Pharmacopoeia monograph for SOMATROPIN FOR INJECTION. The first is a reverse phase HPLC 4method for the determination of related proteins, ie degradation products formed by deamidation and oxidation. The second is a size exclusion HPLC method for determination of dimer and related substances of higher molecular mass.

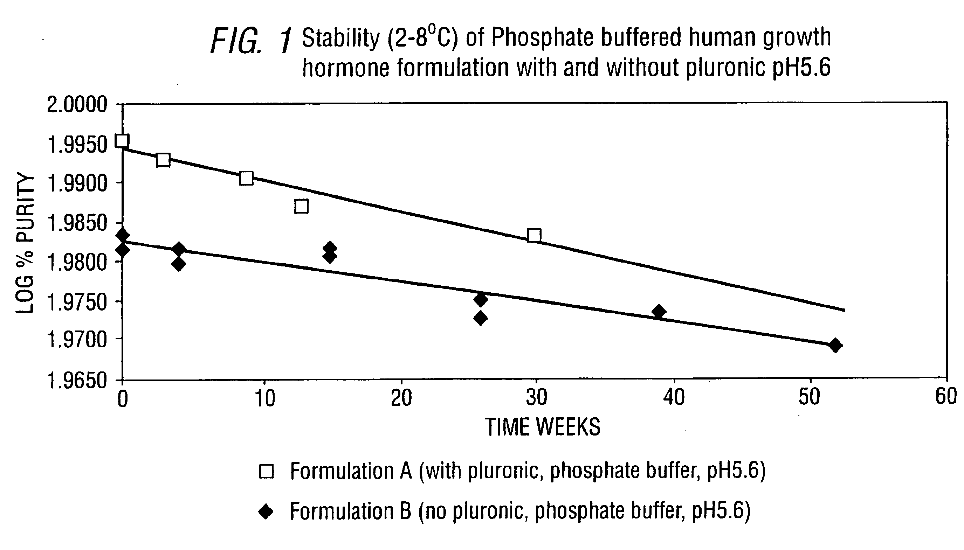

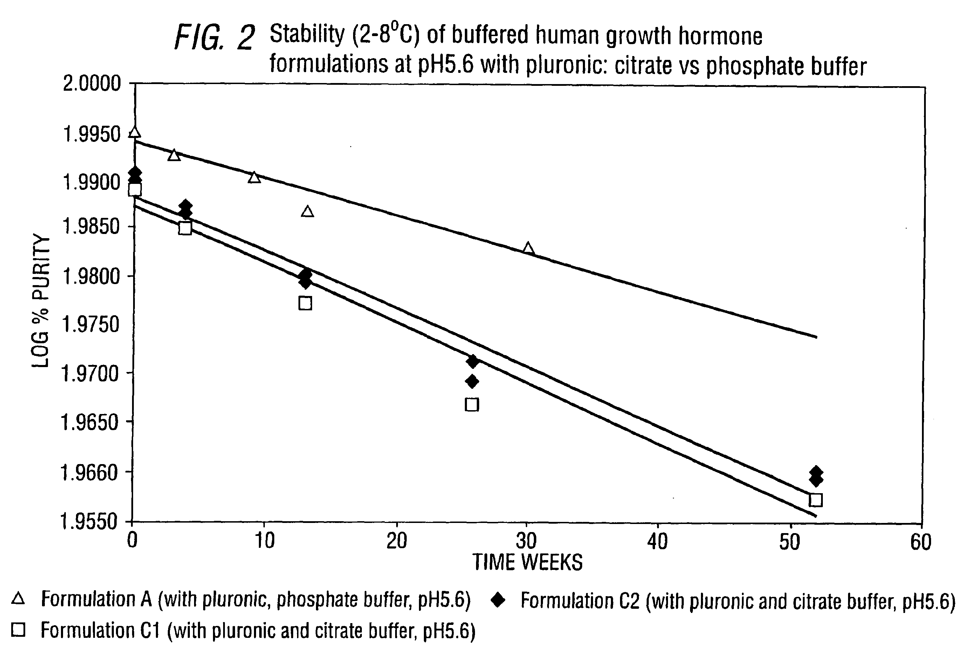

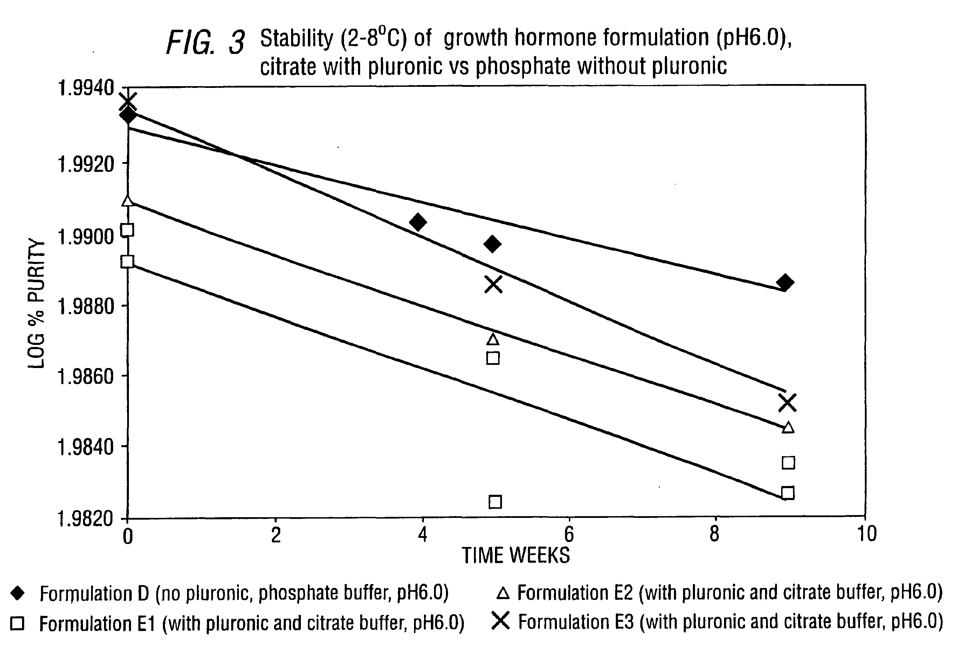

The rpHPLC method was used to ascertain deamidation and oxidation of a number of different formulations over a period of up to 65 weeks stored at 2-8°C. The data is shown in tables 1 to 3 below and graphically in Figures 1 to 3.

Table 4 shows the results of stability studies carried out on Formulation V stored at 2-8°C.

The size exclusion HPLC method referred to above (data not shown) was used to test for aggregation. In no case, during the studies were measurable quantities of dimers and related substances of higher molecular mass found, In all formulations there was less than 1% aggregation (in fact this is the limit of reliable quantitation in the test), ie no aggregation was seen.

The results show clearly that phosphate buffer is better than citrate buffer in terms of stabilising formulations and also that an absence of Pluronic surfactant gives rise to greater stability.

**TABLE 1**

| Stability Study (2-8°C) | | |
|---|---|---|
| Comparative Formulation A (with pluronic, phosphate buffer, pH5.6) | | |
| hGH | 3.33 mg/ml | |
| Pluronic | 0.8 mg/ml | |
| Phosphate buffer | 10mM | |
| Sodium Chloride | 5.9 mg/ml | |
| Benzyl alcohol | 9 mg/ml | |
| | | |

| **Time weeks** | **hGH % purity** | **Log hGH % purity** |
|---|---|---|
| 0 | 98.90 | 1,9952 |
| 3 | 98.35 | 1.9928 |
| 9 | 97.84 | 1.9905 |
| 13 | 97.05 | 1.9870 |
| 30 | 96.26 | 1.9834 |
| k day⁻¹ x 10⁴ | | -1,253 |
| | | |

| Comparative Formulation B (no pluronic, phosphate buffer, pH5.6) | | |
|---|---|---|
| hGH | 3.33 mg/ml | |
| Phosphate buffer | 10mM | |
| Sodium Chloride | 5.9 mg/ml | |
| Benzyl alcohol | 9 mg/ml | |
| | | |

| **Time (wks)** | **hGH % purity** | **log hGH % purity** |
|---|---|---|
| 0 | 96.28 | 1.9835 |
| 0 | 95.88 | 1.9817 |
| 4 | 95.45 | 1.9798 |
| 4 | 95.80 | 1.9814 |
| 15 | 95.67 | 1.9808 |
| 15 | 95.89 | 1.9818 |
| 26 | 94.46 | 1.9752 |
| 26 | 93.94 | 1.9729 |
| 39 | 94.15 | 1.9738 |
| 52 | 93.21 | 1.9695 |
| k day⁻¹ x 10⁴ | | -0.8272 |

**TABLE 2**

| Stability Study (2-8°C) | | |
|---|---|---|
| hGH | 3.33 mg/ml | |
| Pluronic | 0.8 mg/ml | |
| Citrate buffer | 10mM | |
| Sodium Chloride | 5.9 mg/ml | |
| Benzyl alcohol | 9 mg/ml | |
| | | |

| Comparative Formulation C (pH5.6 citrate buffer + pluronic) | | |
|---|---|---|
| | | |
| **time (wks)** | **hGH+** | **log hGH+** |
| 0 | 97.89 | 1,9907 |
| 0 | 97.93 | 1.9909 |
| 4 | 97.12 | 1.9873 |
| 4 | 96.80 | 1.9859 |
| 13 | 95.44 | 1.9797 |
| 13 | 94.85 | 1.9770 |
| 26 | 93.19 | 1.9694 |
| 26 | 93.60 | 1.9713 |
| 52 | 91.32 | 1.9606 |
| 52 | 91.08 | 1.9593 |
| 0 | 97.48 | 1.9889 |
| 0 | 97.71 | 1.9899 |
| 4 | 96.93 | 1,9865 |
| 4 | 96.92 | 1.9864 |
| 13 | 94.89 | 1.9772 |
| 13 | 95.38 | 1.9795 |
| 26 | 92.59 | 1.9666 |
| 26 | 92.65 | 1.9668 |
| 52 | 90.69 | 1.9576 |
| 52 | 91.11 | 1.9596 |
| k day⁻¹ X 10⁴ | | -1.954 |

**TABLE 3**

| Stability Study (2-8°C) | | |
|---|---|---|
| Formulation D | | |
| hGH | 3.33mg/ml | |
| Phosphate buffer | 10mM, pH6.0 | |
| Sodium Chloride | 5.9mg/ml | |
| Benzyl Alcohol | 9mg/ml | |
| | | |

| **Time Weeks** | **hGH % purity** | **log hGH % purity** |
|---|---|---|
| 0 | 98.47 | 1.9933 |
| 4 | 97.82 | 1.9904 |
| 9 | 97.44 | 1.9887 |
| k day⁻¹ X 10⁴ | | -1.65 |

| Stability study (2-8°C) | | |
|---|---|---|
| Comparative Formulation E (citrate buffer pH6.0, with pluronic) | | |
| hGH | 3.33mg/ml | |
| Pluronic | 0.8mg/ml | |
| Citrate buffer | 10mM, pH6.0 | |
| Sodium Chloride | 5.9mg/ml | |
| Benzyl Alcohol | 9mg/ml | |

| **time (wks)** | **hGH % purity** | **log hGH % purity** |
|---|---|---|
| 0 | 97.75 | 1.9901 |
| 0 | 97.56 | 1.9893 |
| 5 | 96.05 | 1.9825 |
| 5 | 96.95 | 1.9865 |
| 9 | 96.29 | 1.9836 |
| 9 | 96.12 | 1.9828 |
| 0 | 97.96 | 1.9910 |
| 0 | 97.93 | 1.9909 |
| 5 | 97.09 | 1.9872 |
| 9 | 96.52 | 1.9846 |
| 9 | 96.51 | 1.9646 |
| 0 | 98.54 | 1.9936 |
| 0 | 98.47 | 1.9933 |
| 5 | 97.68 | 1.9898 |
| 5 | 97.43 | 1.9887 |
| 9 | 96.67 | 1.9853 |
| 9 | 96.77 | 1.9857 |
| k day⁻¹ X 10⁴ | | -2.55 |

**TABLE 4**

| Stability study; Formulation V, 2-8°C | | |
|---|---|---|
| | | |
| **Time Weeks** | **hGH % purity** | **log hGH % purity** |
| 0 | 97.21 | 1.988 |
| 0 | 97.23 | 1.988 |
| 4.5 | 96,50 | 1.985 |
| 4.5 | 96.65 | 1.985 |
| 9 | 95.18 | 1.979 |
| 9 | 95.19 | 1.979 |
| 13 | 95.23 | 1.979 |
| 13 | 95.32 | 1.979 |
| 26 | 94.64 | 1.976 |
| 26 | 94.41 | 1.975 |
| k day⁻¹ X 10⁴ | | -2.489 |

### Example 4 - Avoidance of crystallisation by pH adjustment of liquid formulations

A series of pH variants (0.1 unit increments) of formulation VI were made by adjusting the respective amounts of the phosphate buffer components. 1.5ml aliquots of the formulations were filled into respective capsules for use in pen Injectors. The capsules were stored at 15°C for up to 3 months. The presence or absence of crystals in the capsules was determined by eye over the storage period.

Crystallisation was observed in formulations of below pH 6.2. i.e. at pH 6.1. No crystallisation was observed in formulations of pH 6.2 and above.

By way of comparison, formulation V (pH 6.0) when stored at 15°C or 25°C for up to 6 weeks exhibited crystallisation. Also, formulation V (pH 6.0) exhibited crystallisation in about 2-3 months when stored at 2-8°C.

## Claims

1. A liquid growth hormone formulation comprising human growth hormone in isotonic phosphate buffered solution, a preservative and a non-ionic surfactant, wherein
the isotonicity is provided by a neutral salt, a monosaccharide, a disaccharide or a sugar alcohol,
the pH is in the range of 6.15 to 6.5 and
the liquid formulation has no detectable crystallisation on storage.

2. The formulation as claimed in claim 1, wherein storage is for at least one month, preferably six weeks, more preferably in the range of 1 month to 4 months, most preferably 3 months.

3. The formulation as claimed in claim 1 or 2, wherein the storage temperature is about 2°C or greater, preferably 4°C or greater, more preferably a temperature in the range from 2°C to less than 40°C, even more preferably a temperature in the range from 2°C to 25°C, most preferably 15°C.

4. The formulation as claimed in any of claims 1 to 3, wherein crystallisation is detected by eye, preferably under the light microscope at 5x magnification, more preferably under the light microscope at 10x magnification.

5. The formulation as claimed in any preceding claim, having a pH of 6.2.

6. The formulation as claimed in any preceding claim, wherein isotonicity is provided by mannitol.

7. The formulation as claimed in any preceding claim, wherein the preservative is selected from phenol, benzyl alcohol, meta-cresol, methyl paraben, propyl paraben, benzalkonium chloride and benzethonium chloride.

8. The formulation as claimed in any preceding claim, wherein the non-ionic surfactant is a polysorbate or a poloxamer.

9. The formulation as claimed in claim 8, wherein the non-ionic surfactant is poloxamer 188.

10. The formulation as claimed in any preceding claim, wherein the concentration of the non-ionic surfactant is 0.2 % (w/v).

11. The formulation as claimed in claim 1 in the following composition:

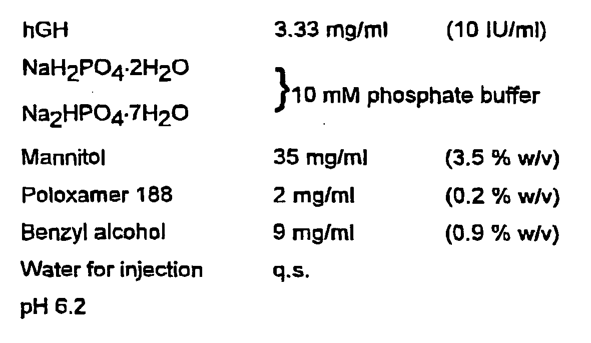

12. The formulation as claimed in claim 11 of the following composition:
| | | |
|---|---|---|
| hGH | 3.33 mg/ml | (10 IU/ml) |
| NaH₂PO₄·2H₂O | 0.85 mg/ml | |
| Na₂HPO₄·7H₂O | 0.31 mg/ml | |
| Mannitol | 35 mg/ml | (3.5 % w/v) |
| Poloxamer 188 | 2 mg/ml | (0.2 % w/v) |
| Benzyl alcohol | 9 mg/ml | (0.9 % w/v) |
| Water for injection | q.s. | |
| pH 6.2 | | |

13. A device for administering a liquid to a human subject by injection and loaded for use with at least one dosage unit of the liquid growth hormone formulation of any of claims 1 to 12.

14. A device as claimed in claim 13 being a pen injector device.

15. A kit comprising an injection device and a separate container of a formulation of any claims 1 to 12.

16. The kit as claimed in claim 15, wherein the container is adapted to engage with the injection device such that in use the formulation in the container is in fluid connection with the outlet of the injection device.

17. The kit as claimed in claim 16, wherein the injection device is a pen injector and the container is a cartridge.

18. A cartridge containing a liquid formulation of any of claims 1 to 12 for use with a pen injector device.

## Patentansprüche

1. Flüssige Formulierung eines Wachstumshormons, umfassend ein humanes Wachstumshormon in einer isotonischen mit Phosphat gepufferten Lösung, ein Konservierungsmittel und ein nicht ionisches Tensid, worin
für die Isotonie durch ein Neutralsalz, ein Monosaccharid, ein Disaccharid oder einen Zuckeralkohol gesorgt wird,
der pH Wert im Bereich von 6,15 bis 6,5 liegt und
die flüssige Formulierung keine detektierbare Kristallisation bei einer Aufbewahrung zeigt.

2. Formulierung nach Anspruch 1, worin die Aufbewahrung wenigstens einen Monat, vorzugsweise sechs Monate, stärker bevorzugt 1 bis 4 Monate, und am meisten bevorzugt 3 Monate beträgt.

3. Formulierung nach Anspruch 1 oder 2, worin die Aufbewahrungstemperatur etwa 2°C oder darüber, vorzugsweise 4°C oder darüber, bevorzugter 2°C bis weniger als 40°C, noch stärker bevorzugt 2°C bis 25°C, und am meisten bevorzugt 15°C beträgt.

4. Formulierung nach einem der Ansprüche 1 bis 3, worin die Kristallisation durch das Auge, vorzugsweise durch ein Lichtmikroskop bei fünffacher Vergrößerung, und bevorzugter durch ein Lichtmikroskop bei zehnfacher Vergrößerung.

5. Formulierung nach einem der vorhergehenden Ansprüche, die einen pH Wert von 6,2 aufweist.

6. Formulierung nach einem der vorhergehenden Ansprüche, worin für die Isotonie durch Mannitol gesorgt wird.

7. Formulierung nach einem der vorhergehenden Ansprüche, worin das Konservierungsmittel ausgewählt ist aus Phenol, Benzylalkohol, meta-Kresol, Methylparaben, Propylparaben, Benzalkoniumchlorid und Benzethoniumchlorid.

8. Formulierung nach einem der vorhergehenden Ansprüche, worin das nicht ionische Tensid ein Polysorbat oder ein Poloxamer ist.

9. Formulierung nach Anspruch 8, worin das nicht ionische Tensid Poloxamer 188 ist.

10. Formulierung nach einem der vorhergehenden Ansprüche, worin die Konzentration des nicht ionischen Tensids 0,2 % (Gew.Nol.) beträgt.

11. Formulierung nach Anspruch 1 mit der folgenden Zusammensetzung:

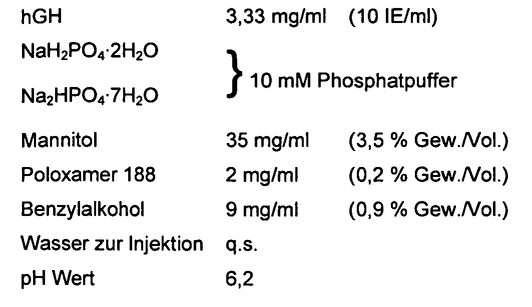

12. Formulierung nach Anspruch 11 mit der folgenden Zusammensetzung:
| | | |
|---|---|---|
| hGH | 3,33 mg/ml | (10 IE/ml) |
| NaH₂PO₄·2H₂O | 0,85 mg/ml | |
| Na₂HPO₄·7H₂O | 0,31 mg/ml | |
| Mannitol | 35 mg/ml | (3,5 % Gew./Vol.) |
| Poloxamer 188 | 2 mg/ml | (0,2 % Gew./Vol.) |
| Benzylalkohol | 9 mg/ml | (0,9 % Gew./Vol.) |
| Wasser zur Injektion | q.s. | |
| pH Wert | 6,2 | |

13. Gerät zur Verabreichung einer Flüssigkeit an einen Menschen durch Injektion, wobei dieses Gerät für die Verwendung mit wenigstens einer Dosierungseinheit der flüssigen Formulierung des Wachstumshormons nach einem der Ansprüche 1 bis 12 beladen ist.

14. Gerät nach Anspruch 13, das ein Injektionspen ist.

15. Bausatz, umfassend ein Injektionsgerät und ein getrenntes Behältnis mit einer Formulierung nach einem der Ansprüche 1 bis 12.

16. Bausatz nach Anspruch 15, worin das Behältnis so beschaffen ist, dass es mit dem Injektionsgerät derart zusammenwirkt, dass beim Gebrauch die Formulierung im Behältnis in Flüssigkeitsverbindung mit dem Auslass des Injektionsgeräts steht.

17. Bausatz nach Anspruch 16, worin das Injektionsgerät ein Injektionspen und das Behältnis eine Patrone ist.

18. Patrone, die eine flüssige Formulierung nach einem der Ansprüche 1 bis 12 enthält, zur Verwendung mit einem Injektionspen.

## Revendications

1. Préparation liquide contenant une hormone de croissance comprenant une hormone de croissance humaine dans une solution isotonique tamponnée par phosphate, un conservateur et un tensioactif non ionique, **caractérisée en ce que**
l'isotonicité est garantie par un sel neutre, un monosaccharide, un disaccharide ou un polysaccharide, **en ce que**
le pH est compris dans la gamme allant de 6,15 à 6,5 et **en ce que**
la préparation liquide ne se cristallise pas lors du stockage.

2. Préparation selon la revendication 1, **caractérisée en ce que** le stockage dure au moins 1 mois, de préférence six semaines, de préférence encore de 1 à 4 mois, plus particulièrement 3 mois.

3. Préparation selon la revendication 1 ou 2, **caractérisée en ce que** la température de stockage est comprise entre 2°C ou plus, de préférence entre 4°C ou plus, de préférence, elle est comprise dans la gamme allant de 2°C à une température inférieure à 40°C, de préférence encore, elle est comprise dans la gamme allant de 2°C à 25°C, plus particulièrement elle est de 15°C.

4. Préparation selon l'une quelconque des revendications 1 ou 3, **caractérisée en ce que** la cristallisation est détectée à l'oeil nu, de préférence à l'aide d'un microscope optique avec un grossissement 5x, de préférence encore à l'aide d'un microscope optique avec un grossissement 10x.

5. Préparation selon l'une quelconque des revendications précédentes présentant un pH de 6,2.

6. Préparation selon l'une quelconque des revendications précédentes, **caractérisée en ce que** l'isotonicité est garantie par du mannitol.

7. Préparation selon l'une quelconque des revendications précédentes, **caractérisée en ce que** le conservateur est sélectionné à partir du phénol, d'alcool benzylique, de méta-crésol, de méthyl parabène, de propyl parabène, de chlorure de benzalkonium et de chlorure de benzéthonium.

8. Préparation selon l'une quelconque des revendications précédentes, **caractérisée en ce que** le tensioactif non ionique est un polysorbate ou un poloxamère.

9. Préparation selon l'une quelconque des revendications précédentes, **caractérisée en ce que** le tensioactif non ionique est le poloxamère 188.

10. Préparation selon l'une quelconque des revendications précédentes, **caractérisée en ce que** la concentration du tensioactif non ionique est de 0,2 % (p/v).

11. Préparation selon la revendication 1 possédant la composition suivante:
| | | |
|---|---|---|
| hGH | 3,33 mg/ml | (10 IU/ml) |
| NaH₂PO₄2H₂O | | |
| | 10 mM de tampon phosphate | |
| NaH₂PO₄7H₂O | | |
| Mannitol | 35 mg/ml | (3,5% p/v) |
| Poloxamer 188 | 2 mg/ml | (0,2% p/v) |
| Alcool benzylique | 9 mg/ml | (0,9% p/v) |
| Eau pour injection | q.s. | |
| pH 6,2 | | |

12. Préparation selon la revendication 11 possédant la composition suivante :
| | | |
|---|---|---|
| hGH | 3,33 mg/ml | (10 IU/ml) |
| NaH₂PO₄·2H₂O | 0,85 mg/ml | |
| NaH₂PO₄·7H₂O | 0,31 mg/ml | |
| Mannitol | 35 mg/ml | (3,5% p/v) |
| Poloxamer 188 | 2 mg/ml | (0,2% p/v) |
| Alcool benzylique | 9 mg/ml | (0,9% p/v) |
| Eau pour injection | q.s. | |
| pH 6,2 | | |

13. Dispositif d'administration d'un liquide à un sujet humain par injection et chargé en vue d'être utilisé avec au moins une unité de dosage de la préparation liquide contenant l'hormone de croissance selon l'une quelconque des revendications 1 à 12.

14. Dispositif selon la revendication 13 se présentant sous forme d'un stylo injecteur.

15. Kit comprenant un dispositif d'injection et un conteneur séparé contenant une préparation selon l'une quelconque des revendications 1 à 12.

16. Kit selon la revendication 15, **caractérisé en ce que** le conteneur est conçu de façon à s'engager dans le dispositif d'injection de façon à ce que la préparation liquide contenue dans le conteneur soit en connexion fluide avec la sortie du dispositif d'injection.

17. Kit selon la revendication 16, **caractérisé en ce que** le dispositif d'injection est un stylo injecteur et **en ce que** le conteneur est une cartouche.

18. Cartouche contenant une préparation liquide selon l'une quelconque des revendications 1 à 12 utilisable avec un stylo injecteur.
